# EUROPEAN PATENT APPLICATION

(11) **EP 4 678 750 A1**
(43) Date of publication of application: **14.01.2026**
(21) Application number: 24788711.0
(22) Date of filing: 09.04.2024
(51) Int. Cl.: C12N 15/50, C12N 15/63, C12Q 1/02

(54) **REPLICON DNA, CLONING VECTOR, SCREENING KIT, SCREENING METHOD, ANTI-NOBECOVIRUS AGENT, BETA-CORONAVIRUS PROLIFERATION INHIBITOR, PAN BETA-CORONAVIRUS INHIBITOR, VIRUS PROLIFERATION INHIBITOR, HEPATITIS C VIRUS PROLIFERATION INHIBITOR, AND METHOD FOR SCREENING THERAPEUTIC AGENT FOR VIRAL INFECTIOUS DISEASE**

(30) Priority: 11.04.2023 JP 2023064237
(71) Applicant: Kagoshima University, Kagoshima-shi, Kagoshima 890-8580 (JP)
(72) Inventor: IKEDA Masanori, Kagoshima-shi, Kagoshima 890-8580 (JP); TAKEDA Midori, Kagoshima-shi, Kagoshima 890-8580 (JP); LI Jiazhou, Kagoshima-shi, Kagoshima 890-8580 (JP)
(74) Representative: v. Bezold & Partner Patentanwälte - PartG mbB
(86) International application number: PCT/JP2024/014354
(87) International publication number: WO 2024/214687

(57) **Abstract**

The replicon DNA includes a CMV promoter sequence that functions in vertebrate cells, a non-structural region containing nobecovirus non-structural protein genes 1a and 1b located on the 3' side of the CMV promoter sequence, and a nobecovirus N gene located on the 3' side of the non-structural region.

## Description

### Technical Field

The present disclosure relates to a replicon DNA, a cloning vector, a screening kit, a screening method, an anti-nobecovirus agent, a betacoronavirus proliferation inhibitor, a pan-betacoronavirus inhibitor, a viral proliferation inhibitor, a hepatitis C virus proliferation inhibitor, and a screening method for a therapeutic agent for a viral infectious disease.

### Background Art

Coronaviruses that are highly pathogenic to humans, including Severe Acute Respiratory Syndrome Coronavirus 2 (SARS-CoV-2), which causes severe acute respiratory syndrome (SARS), and Middle East Respiratory Syndrome Coronavirus (MERS-CoV), all belong to the Betacoronavirus family. Five subtypes of Betacoronavirus are known: Sarbecovirus, Hibecovirus, Nobecovirus, Merbecovirus, and Embecovirus. SARS-CoV-2 and Coronavirus SARS-CoV-1, which was known before SARS-CoV-2, are classified as Sarbecoviruses. MERS-CoV is classified as a Merbecovirus.

Patent Literature 1 discloses a replicon DNA including a non-structural region containing SARS-CoV-2 non-structural protein genes 1a and 1b and a SARS-CoV-2 N gene. The viral replicon DNA maintains the non-structural region, so as to be able to perform autonomous replication, but lacks the structural region, so as to lose its infectivity. Since the replicon DNA lacking infectivity can be handled even in facilities with BSL of 2, the development of anti-SARS-CoV-2 agents is expected to be promoted.

### Citation List

### Patent Literature

Patent Literature 1: International Publication No. WO 2022/158067

### Summary of Invention

### Technical Problem

Among the Betacoronaviruses, Nobecovirus and Hibekovirus have not been reported to infect humans. As for Nobecovirus and Hibekovirus, they are candidates for the next outbreak and require attention and countermeasures.

The present disclosure was made in view of the above-mentioned circumstances, and an object of the present disclosure is to provide a replicon DNA, a cloning vector, a screening kit and a screening method, which enable easy screening for an anti-nobecovirus agent. Furthermore, another object of the present disclosure is to provide a novel anti-nobecovirus agent, a novel betacoronavirus proliferation inhibitor, a novel pan-betacoronavirus inhibitor, a novel viral proliferation inhibitor, a novel hepatitis C virus proliferation inhibitor and a novel screening method for a therapeutic agent for a viral infectious disease.

### Solution to Problem

### Replicon DNA

The replicon DNA described herein includes
a promoter sequence that functions in vertebrate cells,
a non-structural region containing nobecovirus non-structural protein genes 1a and 1b located on the 3' side of the promoter sequence, and
a nobecovirus N gene located on the 3' side of the non-structural region.

The replicon DNA described herein may further include a reporter gene.

### Cloning vector

The cloning vector described herein includes the replicon DNA described herein.

### Screening kit

The screening kit described herein is provided with:
the cloning vector described herein, and
hepatitis C virus-cured cells.

### Screening method

The screening method described herein includes:
exposing hepatitis C virus-cured cells transfected with the cloning vector described herein, to a test substance;
quantifying a replicon RNA replicated from a replicon RNA transcribed from the replicon DNA in the hepatitis C virus-cured cells; and
selecting a test substance that inhibits the replication of the replicon RNA more than the control.

### Anti-nobecovirus agent

The anti-nobecovirus agent described herein includes a compound selected from the group consisting of navitoclax, ABT-737, A-1155463, gambogic acid, A-1331852, WEHI-539, toll-like receptor modulator, CC-401, Y-27632, AZD7545, CB-839, etomoxir, FK-866, daurisoline, dauricine, demethoxycurcumin, and pharmacologically acceptable salts thereof.

The anti-nobecovirus agent described herein may be a nobecovirus proliferation inhibitor.

### Betacoronavirus proliferation inhibitor

The betacoronavirus proliferation inhibitor described herein contains
a Bcl-xL inhibitor or a Bcl-xL expression inhibitor.

The betacoronavirus proliferation inhibitor described herein contains a compound selected from the group consisting of ABT-737, A-1155463, gambogic acid, A-1331852, WEHI-539, and pharmacologically acceptable salts thereof.

The betacoronavirus may be one or more types of betacoronaviruses selected from the group consisting of Sarbecovirus, Merbecovirus, Nobecovirus, and Embecovirus.

### Pan-betacoronavirus inhibitor

The pan-betacoronavirus inhibitor described herein contains a Bcl-xL expression inhibitor or a Bcl-xL inhibitor.

### Viral proliferation inhibitor

The viral proliferation inhibitor described herein contains a Bcl-xL expression inhibitor or a Bcl-xL inhibitor.

### Hepatitis C virus proliferation inhibitor

The hepatitis C virus proliferation inhibitor described herein contains a Bcl-xL expression inhibitor or a Bcl-xL inhibitor.

### Screening method for therapeutic agent for viral infectious disease

The screening method for a therapeutic agent for a viral infectious disease described herein includes:
measuring the Bcl-xL expression inhibitory activity or the Bcl-xL inhibitory activity of a test substance; and
selecting the test substance having the Bcl-xL expression inhibitory activity or the Bcl-xL inhibitory activity as a proliferation inhibitor for the of two or more types of betacoronaviruses selected from the group consisting of Sarbecovirus, Merbecovirus, Nobecovirus and Embecovirus, a pan-betacoronavirus inhibitor, a viral proliferation inhibitor or a hepatitis C virus proliferation inhibitor.

### Advantageous Effects of Invention

According to the present disclosure, an anti-nobecovirus agent can be conveniently screened for. Further, according to the present disclosure, a novel anti-nobecovirus agent, a novel betacoronavirus proliferation inhibitor, a novel pan-betacoronavirus inhibitor, a novel viral proliferation inhibitor, a novel hepatitis C virus proliferation inhibitor and a novel screening method for a therapeutic agent for a viral infectious disease are provided.

### Brief Description of Drawings

FIG. 1A is a diagram illustrating a nobecovirus genome composition;
FIG. 1B is a diagram illustrating an example of a replicon DNA composition and a fragment designed based on the nucleotide sequence of the replicon DNA;
FIG. 2 is a diagram illustrating the replication level of each replicon RNA according to Example 1;
FIG. 3A is a diagram illustrating the concentration responsiveness to navitoclax of the replication level of the nobecovirus replicon RNA;
FIG. 3B is a diagram illustrating cell viability against navitoclax;
FIG. 4 is a diagram illustrating the replication level of each replicon RNA in the presence of navitoclax and cell viability;
FIG. 5 is a diagram illustrating the replication levels of nobecovirus replicon RNA and SARS-CoV-2 replicon RNA in the presence of compounds and cell viability;
FIG. 6 is a diagram illustrating the replication levels of SARS-CoV-2 replicon RNA and nobecovirus replicon RNA in the presence of natural products, and cell viability;
FIG. 7 is a diagram illustrating the replication levels of nobecovirus replicon RNA in the presence of natural products;
FIG. 8 is a diagram illustrating the replication levels of SARS-CoV-2 replicon RNA in the presence of the inhibitors of proteins belonging to the Bcl-2 family and cell viability;
FIG. 9 is a diagram illustrating the replication level of each replicon RNA in the presence of Bcl-xL inhibitors and cell viability;
FIG. 10A is a diagram illustrating the A-1155463 concentration responsiveness of the replication level of SARS-CoV-2 replicon RNA;
FIG. 10B is a diagram illustrating cell viability in the presence of A-1 155463;
FIG. 11 is a diagram illustrating the replication level of each replicon RNA in the presence of Bcl-xL inhibitors and cell viability;
FIG. 12 is a diagram illustrating the Bcl-xL selective inhibitor A-1331852 concentration responsiveness of the replication levels of SARS-CoV-2 replicon RNA;
FIG. 13 is a diagram illustrating the replication level of each replicon RNA in the presence of existing drugs against COVID-19 and cell viability;
FIG. 14 is a diagram illustrating the replication levels of SARS-CoV-2 replicon RNA in cells wherein the expression of genes belonging to the Bcl-2 family was inhibited;
FIG. 15A is a diagram illustrating the composition of hepatitis C virus (hereinafter, also referred to as "HCV") subgenomic replicon according to Example 2; and
FIG. 15B is a diagram illustrating the A-1331852 concentration responsiveness of the replication level of HCV replicon RNA.

### Description of Embodiments

The embodiments according to the present disclosure are described with reference to the drawings. The present disclosure is not limited by the following embodiments and drawings. In the following embodiments, expressions such as "having", "including", or "containing" also include the meanings of "comprising" or "consisting of'.

### Embodiment 1: Replicon DNA

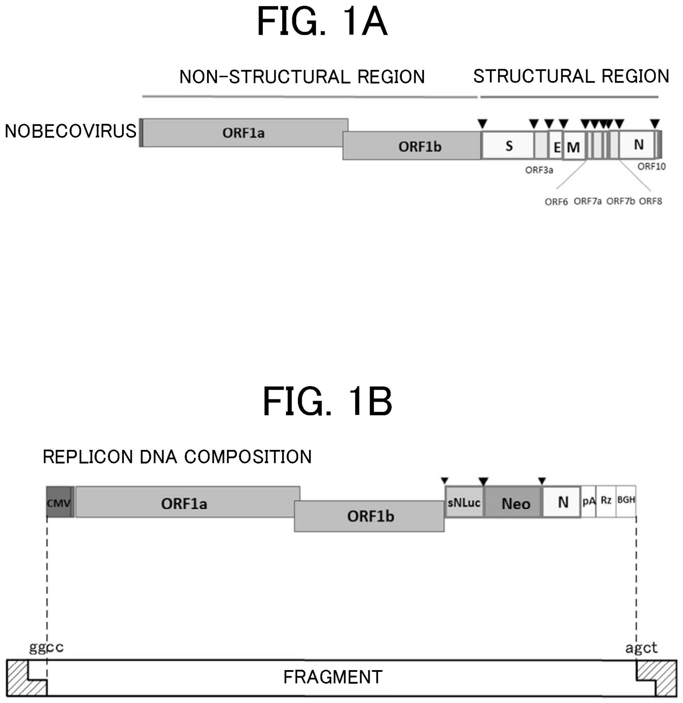

FIG. 1A illustrates the genome composition of Nobecovirus. The Nobecovirus genome has a non-structural region containing a non-structural protein gene 1a (ORF1a) and a non-structural protein gene 1b (ORF1b) and a structural region containing an S gene, ORF3a, an E gene, an M gene, ORF6, ORF7a, ORF7b, ORF8, an N gene and ORF10. Untranslated regions (5' UTR and 3' UTR) are present on the 5' and 3' ends of the Nobecovirus genome, respectively. The nucleotide sequence of the Nobecovirus genome, for example, is registered in the database of the National Center for Biotechnology Information (NCBI) (NCBI Reference Sequence: NC_009021).

The replicon DNA according to the present embodiment is a DNA construct constructed based on the nucleotide sequence of the Nobecovirus genome. In general, viral replicons maintain a non-structural region and lack a structural region, so these are not infectious but can replicate autonomously. FIG. 1B illustrates an example of the composition of the replicon DNA according to the present embodiment. The replicon DNA has the Nobecovirus non-structural region but does not have any gene contained in the structural region except for the N gene. Specifically, the replicon DNA has a promoter sequence, a non-structural region containing ORF1a and ORF1b, and the N gene.

The promoter sequence is a promoter sequence that functions in vertebrate cells. Preferably, the promoter sequence is located at the 5' end of the replicon DNA. Examples ofthe promoter sequence include a cytomegalovirus (CMV) promoter illustrated as "CMV" in FIG. 1B, an SV40 promoter, a retrovirus promoter, a metallothionein promoter, a heat shock protein promoter, a CAG promoter, an elongation factor 1α promoter, an actin promoter, a ubiquitin promoter, an albumin promoter, and an MHC class promoter.

The non-structural region is located on the 3' side of the promoter sequence. The N gene located on the 3' side of the non-structural region encodes a nucleoprotein. The nucleoprotein binds to the genomic RNA of Nobecovirus and forms a helical nucleocapsid. The N gene improves the replication level of genomic RNA.

The replicon DNA according to the present embodiment is transcribed into RNA (replicon RNA) within the cell, then translated and replicated. The replicon DNA may have a reporter gene to quantify the replicated replicon RNA. Any known reporter gene can be used as the reporter gene, and examples thereof include a gene encoding a green fluorescent protein, a gene encoding β-glucuronidase, and a gene encoding luciferase. Preferably, the reporter gene is a secreted luciferase gene (sNLuc).

The replicon DNA may also contain a marker gene, if necessary. The marker gene is not particularly limited, and may be, for example, a drug resistance gene. Examples of the drug resistance gene include a neomycin resistance gene (Neo), a hygromycin resistance gene, and a puromycin resistance gene.

The replicon DNA may also contain other sequences used in gene expression systems. Examples of other sequences include a poly(A) region (pA) that contributes to the stability of the transcript, a Hepatitis Delta Virus (HDV) ribozyme gene (Rz) that is necessary for excision by self-cleavage after replication, and a bovine growth hormone poly(A) signal (BGH) that terminates transcription. Preferably, the replicon DNA according to the present embodiment has sNLuc and Neo between ORF 1b and the N gene, and has pA, Rz, and BGH on the 3' side of the N gene, as illustrated in FIG. 1B. The nucleotide sequence of the replicon DNA having the composition illustrated in FIG. 1B is exemplified by SEQ ID NO: 1.

The replicon DNA according to the present embodiment is chemically synthesized according to its nucleotide sequence by a known method such as any nucleic acid synthesis method, including, for example, a solid-phase phosphoramidite method. Various automated nucleotide synthesizers are commercially available, and replicon DNA can also be synthesized using such automated nucleotide synthesizers. A multinucleotide synthesis method can also be used as appropriate.

Below, a suitable production method for producing the replicon DNA according to the present embodiment is described. This production method uses a type IIS restriction enzyme (Golden Gate method). The type IIS restriction enzyme is an enzyme that cleaves at least one of the two strands at a site downstream of the recognition site. Examples of the type IIS restriction enzyme include *Alw*I, *Alw*XI, *Alw*26I, *Bbs*I, *Bbv*I, *Bbv*II, *Bce*fI, *Bcc*I*, Bcg*I, *Bci*VI, *Bin*I, *Bmr*I, *Bpm*I, *Bsa*I, *Bse*RI, *Bsg*I, *Bsm*AI, *Bsm*BI, *BspMI, BsrDI, Bst*F5I, *Ear*I, *Eco*31I, *Eco*57I, *Esp*3I, *Esp*3I, *Fau*I, *Fok*I, *Gsu*I, *Hga*I, *HinGUII, Hphl, Ksp632I, Mbo*II, *Mme*I, *Mn1*I, *Ngo*VIII, *Ple*I, *Psr*I, *Rle*AI, *Sap*I, *Sfa*NI, *Taq*II, *Tth*111II, *Bsm*I, *Bsr*I, *Bsm*FI and *Bse*MII.

In the following, *Bsa*I is used as the type IIS restriction enzyme. *Bsa*I cleaves at a site downstream of the recognition sequence, allowing the design of the nucleotide sequence of the 4-base protruding end. Since the *Bsa*I recognition sequence does not remain in the DNA fragment after cleavage, replicon DNA can be constructed using the 4-base protruding end.

First, as illustrated in FIG. 1B, a fragment is defined in which the nucleotide sequence of the *Bsa*I site is added at both ends of the replicon DNA.

Preferably, in the replicon DNA, the recognition site for the type IIS restriction enzyme is synonymously substituted. Specifically, the replicon DNA does not have a recognition site for the type IIS restriction enzyme used in producing the replicon DNA. The synonymous substitution is a base substitution that does not change amino acids to be encoded, and is also called a silent mutation. The synonymous substitution of the recognition site for the type IIS restriction enzyme makes it possible to avoid cleavage of fragments at unnecessary sites when the type IIS restriction enzyme is acted upon during the production process.

The fragment is then introduced into a plasmid vector (nucleic acid construct). Any plasmid vector may be used, but preferably it is a plasmid vector that does not have a *Bsa*I site. When using a plasmid vector having a *Bsa*I site, the *Bsa*I site must be deleted by synonymous substitution or the like of the nucleotide sequence before use.

Next, the fragment is introduced into the cloning site of a cloning vector having *Bsa*I sites only at both ends of the cloning site. Specifically, the method for producing a cloning vector includes a cleavage step and a ligation step. In the cleavage step, the above plasmid vector and a cloning vector having *Bsa*I sites only at both ends of the cloning site are cleaved at the *Bsa*I sites. In the ligation step, the fragment excised in the cleavage step is ligated to the cloning vector using DNA ligase, thereby inserting the replicon DNA into the cloning site. Preferably, cloning vector D is BAC.

The obtained cloning vector is transduced into *E. coli* or the like by known methods, and the transduced clones are selected by known colony screening.

The replicon DNA according to the present embodiment is introduced into mammalian cells using known methods, for example, via a cloning vector, and thus can replicate autonomously within the cells. This enables the evaluation or the like of compounds that inhibit Nobecovirus replication.

The replicon DNA according to the present embodiment can be produced without using natural viruses as templates, which can avoid infection, spillage accidents, and the like. In addition, the replicon DNA can be produced and used in facilities with BSL of less than 3. Furthermore, because the replicon DNA has a promoter sequence that functions in vertebrate cells, it can be directly introduced as DNA into mammalian cells and replicated. This eliminates the need for *in vitro* transcription prior to introduction into cells, and also eliminates the need to introduce RNA into cells, which is cumbersome and relatively difficult to manipulate. From the above, the replicon DNA according to the present embodiment has a higher transfection efficiency than direct transfection of replicon RNA into cells, and is therefore highly practical.

The replicon DNA according to the present embodiment may further have a reporter gene. This allows the replication level to be assessed via the reporter gene. Further in the replicon DNA, the recognition sites for the type IIS restriction enzyme may be synonymously substituted. Since the replicon DNA does not have a recognition site for the type IIS restriction enzyme, the replicon DNA can be efficiently constructed using the type IIS restriction enzyme in the above method for producing replicon DNA.

Alternatively, the above fragment may be divided into multiple partial fragments, and each partial fragment may be ligated and introduced into a cloning vector.

In another embodiment, a cloning vector having the replicon DNA according to the present embodiment is provided.

In another embodiment, a nobecovirus is provided. The nobecovirus may have a non-structural protein selected from the following (a) to (c):
(a) a non-structural protein comprising the amino acid sequence represented by SEQ ID NO: 2;
(b) a non-structural protein comprising an amino acid sequence obtained by substitution, deletion, insertion or addition of 1 to 5 amino acids in the amino acid sequence represented by SEQ ID NO: 2, and having a function of replicating the nobecovirus genome; and
(c) a non-structural protein comprising an amino acid sequence having 90% or more sequence identity with the amino acid sequence represented by SEQ ID NO: 2, and having a function of replicating the nobecovirus genome.

Further, the nobecovirus may have a non-structural protein selected from the following (d) to (f);
(d) a non-structural protein comprising the amino acid sequence represented by SEQ ID NO: 3;
(e) a non-structural protein comprising an amino acid sequence obtained by substitution, deletion, insertion or addition of 1 to 5 amino acids in the amino acid sequence represented by SEQ ID NO: 3, and having a viral protein-specific degrading enzyme function;
(f) a non-structural protein comprising an amino acid sequence having 90% or more sequence identity with the amino acid sequence represented by SEQ ID NO: 3, and having a viral protein-specific degrading enzyme function.

The nobecovirus may also have any of the following nucleoproteins selected from the following (g) to (i):
(g) a nucleoprotein comprising the amino acid sequence represented by SEQ ID NO: 4;
(h) a nucleoprotein comprising an amino acid sequence obtained by substitution, deletion, insertion, or addition of 1 to 5 amino acids in the amino acid sequence represented by SEQ ID NO: 4, and having a function of binding to the nobecovirus genomic RNA and forming a spiral nucleocapsid;
(i) a nucleoprotein comprising an amino acid sequence having 90% or more sequence identity with the amino acid sequence represented by SEQ ID NO: 4, and having a function of binding to the nobecovirus genomic RNA and forming a spiral nucleocapsid.

The number of amino acids to be substituted, deleted, inserted or added as described above is preferably 1 to 4, 1 to 3, 1 to 2 or 1. In the case of substitution, preferably, an acidic amino acid is substituted with any other acidic amino acid. Similarly, a basic amino acid, an aromatic amino acid, an aliphatic amino acid, and an hydroxyamino acid are preferably substituted with another basic amino acid, another aromatic amino acid, another aliphatic amino acid, and another hydroxyamino acid, respectively.

The "sequence identity" between two amino acid sequences is the ratio of the number of identical amino acids corresponding to each other in the two amino acid sequences to the total number of amino acids in the full-length sequences when the two amino acid sequences are aligned. The identity of two amino acid sequences can be determined using known algorithms. The above-mentioned "90% or more sequence identity" means that the sequence homology is at least 90%, and preferably the sequence identity is, for example, 91%, 92%, 93%, 94%, 95%, 96%, 97%, 98% or 99% or more.

### Embodiment 2: Screening kit

The screening kit according to the present embodiment is provided with the cloning vector according to Embodiment 1 and HCV-cured cells. "HCV-cured cells" are cells obtained by culturing cells that replicate a subgenomic HCV replicon or cells that replicate the full-length HCV genome in the presence of a drug having antiviral activity, and are cells from which the subgenomic HCV replicon or the full-length HCV genome has been eliminated. Preferably, HCV-cured cells are free of HCV RNA and HCV proteins expressed in the cells. Whether or not HCV-derived RNA is contained in cells can be easily confirmed by reverse transcription polymerase chain reaction (RT-PCR), Northern blotting, or the like. Whether or not HCV proteins are expressed in cells can be easily confirmed by Western blotting or the like.

HCV-cured cells can be established, for example, by the following method. After introduction of full-length HCV RNA into mammalian cells such as HuH-7 cells by electroporation, G418 is added and thus cells with a high level of HCV RNA replication are selected to form colonies. By adding interferon (IFN) to a cell line of cloned cells with the highest HCV RNA replication level among the colonies, a cured cell line in which HCV RNA has been eliminated can be obtained. The cured cell line is known to have a higher replication level of HCV RNA than the parent cell line.

The screening kit according to the present embodiment may further be provided with a medium, a medium additive, and the like that can be used for culturing HCV-cured cells.

Next, a screening method provided in another embodiment is described as a method for using the screening kit. The screening method is particularly suitable for screening for anti-nobecovirus agents. The screening method includes a transfection step, an exposure step, a quantification step, and a selection step.

In the transfection step, a cloning vector containing the replicon DNA according to Embodiment 1 above is transfected into HCV-cured cells. The cloning vector can be transfected into HCV-cured cells by known methods. For example, the cloning vector is transfected into HCV-cured cells using commercially available transfection reagents, or the like.

In the exposure step, HCV-cured cells transfected with the cloning vector are exposed to a test substance. In the exposure step, the test substance may be added to a medium containing HCV-cured cells. The test substance is not particularly limited and may be any substance such as a compound, a peptide, and a nucleic acid.

In the quantification step, a replicon RNA replicated from a replicon RNA transcribed from the replicon DNA in HCV-cured cells is quantified. The method for quantifying nucleic acids is not particularly limited, and quantitative PCR or the like may be used. In particular, if the replicon DNA has a secreted luciferase gene, the replicated replicon RNA can be quantified by collecting the culture supernatant and measuring the luciferase activity using a commercially available kit or the like. Note that "quantitation" includes not only determining the amount of replicated replicon RNA but also assessing the relative amount.

In the selection step, a test substance that inhibits the replication of replicon RNA more than the control in the quantification step is selected. Controls are, for example, HCV-cured cells that have not been exposed to the test substance. Preferably, the control is a known anti-nobecovirus agent or the like that inhibits Nobecovirus infection or replication. When the replicated nucleic acid is quantified by the magnitude of luciferase activity, the selection step may be performed by comparing the luciferase activity between the control and the test substance. In this case, the test substance whose luciferase activity exceeds that of the control is selected as an anti-nobecovirus agent or a candidate substance thereof.

According to the screening kit according to the present embodiment and the screening method according to another embodiment, HCV-cured cells in which the above replicon RNA is replicated particularly strongly are used, so that test substances that inhibit the replication of replicon RNA can be selected with high accuracy. As described in Example 1 below, the replication of replicon RNA in HCV-cured cells responds to the concentration of a compound having anti-nobecovirus activity, so the concentration dependency of the test substance can also be assessed. In the selection step, the measured value of the test substance may be compared with a reference value set in advance as a reference.

Furthermore, test substances selected by the above screening method can be used as anti-nobecovirus agents. Therefore, the above screening method constitutes at least a part of the method for producing an anti-nobecovirus agent.

### Embodiment 3: Anti-nobecovirus agent

The anti-nobecovirus agent of the present embodiment contains, as an active ingredient, a compound selected from the group consisting of navitoclax, ABT-737, A-1155463, gambogic acid, A-1331852, WEHI-539, toll-like receptor modulator, CC-401, Y-27632, AZD7545, CB-839, etomoxir, FK-866, daurisoline, dauricine, demethoxycurcumin, and pharmacologically acceptable salts thereof. As used herein, the term "anti-nobecovirus agent" refers to a drug that exhibits at least one of a therapeutic effect and a preventive effect against nobecovirus infections. The anti-nobecovirus agent may be a nobecovirus proliferation inhibitor, for example.

The structures of navitoclax, ABT-737, A-1155463, gambogic acid, A-1331852, WEHI-539, toll-like receptor modulator, CC-401, Y-27632, AZD7545, CB-839, etomoxir, FK-866 (dapolinad), daurisoline, dauricine, and demethoxycurcumin are illustrated below.

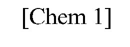

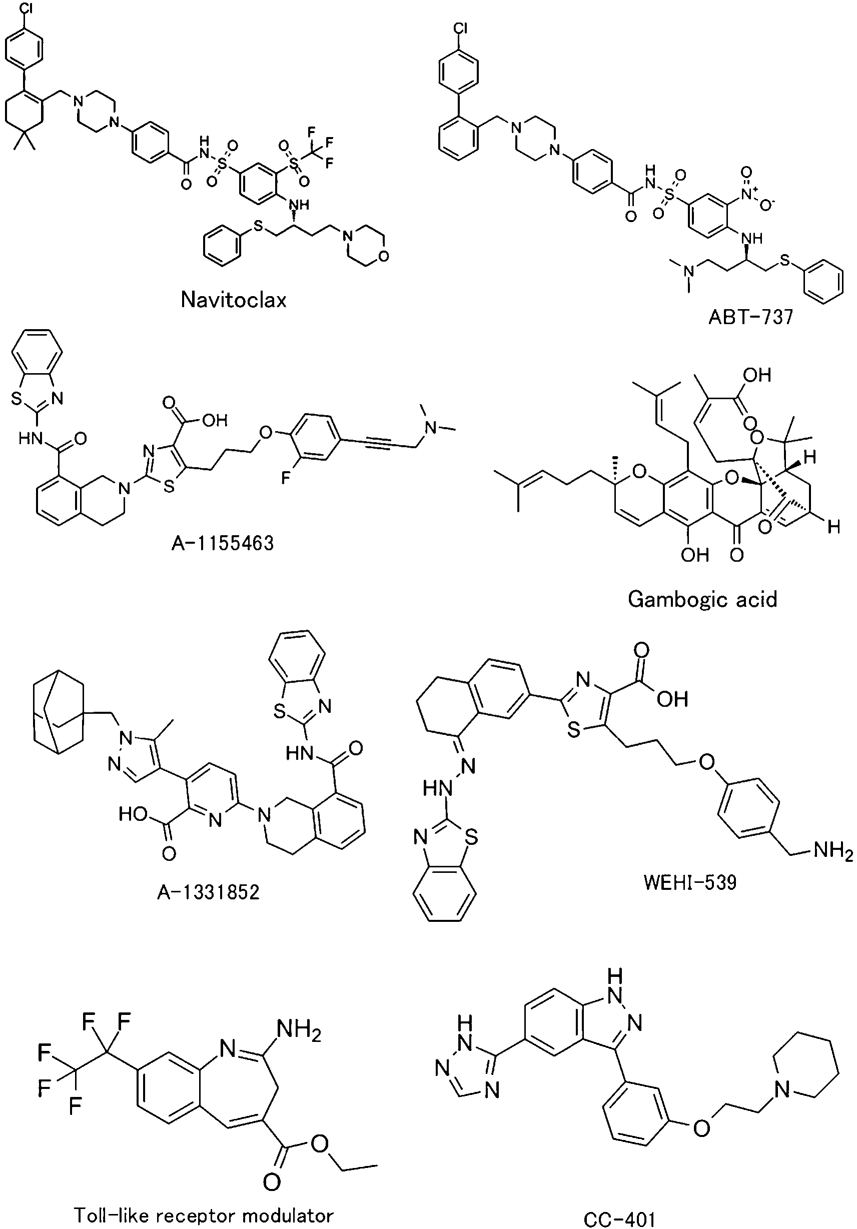

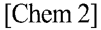

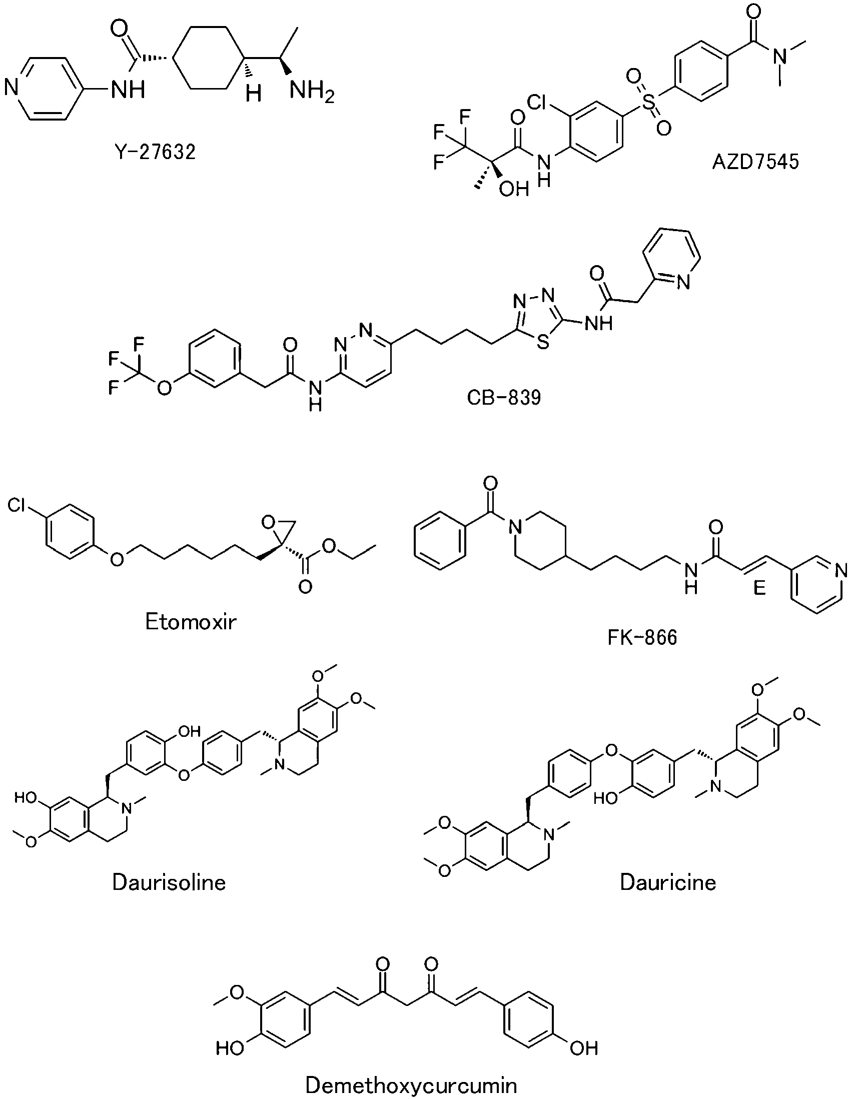

The pharmacologically acceptable salts mentioned above are not particularly limited, and examples thereof include alkali metal salts such as a lithium salt, a sodium salt, and a potassium salt; alkaline earth metal salts such as a magnesium salt and a calcium salt; inorganic acid salts such as hydrochloride, dihydrochloride, hydrobromide, sulfate, nitrate, oxalate, and phosphate; and organic acid salts such as acetate, propionate, hexanoate, cyclopentanepropionate, glycolate, pyruvate, lactate, malonate, succinate, malate, fumarate, tartrate, citrate, benzoate, o-(4-hydroxybenzoyl)benzoate, cinnamate, mandelate, methanesulfonate, ethanesulfonate, 1,2-ethanedisulfonate, 2-hydroxyethanesulfonate, benzenesulfonate, p-chlorobenzenesulfonate, 2-naphthalenesulfonate, p-toluenesulfonate, camphorsulfonate, 4-methylbicyclo[2.2.2]oct-2-ene-1-carboxylate, glucoheptanoate, 3-phenylpropionate, trimethyl acetate, tert-butyl acetate, lauryl sulfate, gluconate, glutamate, hydroxynaphthoate, salicylate, stearate, and muconate. The anti-nobecovirus agent may also contain multiple types of the above compounds as active ingredients. Antiviral activity refers to the activity of reducing the infectivity, proliferation ability, or immune evasion ability of Nobecovirus.

For example, the salts of WEHI-539 and CC-401 are preferably hydrochlorides, and the salt of Y27632 is preferably dihydrochloride.

The anti-nobecovirus agent according to the present embodiment is produced by a known method and contains, as an active ingredient, 0.000001% to 99.9% by weight, 0.00001% to 99.8% by weight, 0.0001% to 99.7% by weight, 0.001% to 99.6% by weight, 0.01% to 99.5% by weight, 0.1% to 99% by weight, 0.5% to 60% by weight, 1% to 50% by weight, or 1% to 20% by weight of the above compound. The anti-nobecovirus agent may be a solid or liquid formulation.

In addition to the above compounds, the anti-nobecovirus agent may also contain any pharmacologically acceptable ingredient. Examples of such optional ingredients include carriers, excipients, lubricants, binders, disintegrants, solvents, solubilizers, suspending agents, isotonicity agents, buffers, and soothing agents. If necessary, additives such as preservatives, antioxidants, coloring agents, and sweeteners may be added to the anti-nobecovirus agent.

The dosage of the anti-nobecovirus agent according to the present embodiment is appropriately determined according to the sex, age, body weight, symptoms and the like of the subject. The anti-nobecovirus agent is administered in such a manner that the amount of the above compounds is an effective amount. The effective amount is the amount of the compound(s) required to achieve the desired result, that is, to inhibit the proliferation of Nobecovirus or to delay, inhibit, prevent, reverse, or cure the progression of symptoms caused by Nobecovirus infection.

The dosage of the anti-nobecovirus agent is, for example, 0.01 mg/kg to 1,000 mg/kg, preferably 0.1 mg/kg to 200 mg/kg, more preferably 0.2 mg/kg to 20 mg/kg, and can be administered once a day or in more divided doses. When the anti-nobecovirus agent is administered in divided doses, the anti-nobecovirus agent is administered 2 to 5 times a day. The anti-nobecovirus agent may be administered at various dosing frequencies, such as daily, every other day, once a week, every other week, and once a month. Amounts outside the above range can also be used if necessary.

The route of administration of the anti-nobecovirus agent is not particularly limited. The anti-nobecovirus agent may be administered, for example, parenterally or orally. Parenteral administration may be intravenous injection, subcutaneous injection, intraperitoneal injection, intramuscular injection, transdermal administration, intranasal administration, transpulmonary administration, enteral administration, and transmucosal administration, for example. The anti-nobecovirus agent may be administered via intravenous infusion.

The anti-nobecovirus agent can be formulated in any form. For oral administration, the anti-nobecovirus agent may be in the form of tablets such as sugar-coated tablets, buccal tablets, coated tablets and chewable tablets, troches, pills, dispersions and capsules including soft capsules, granules, suspensions, emulsions and syrups including dry syrup, and liquids such as elixirs. For parenteral administration, the anti-nobecovirus agent may be administered in the form of injections, inhalants, transdermal patches, aerosols, suppositories, and the like.

The anti-nobecovirus agent can be administered to any subject as long as the subject is infected with nobecovirus. For example, the subject to which the anti-nobecovirus agent is administered is preferably a vertebrate, more preferably a mammal. Examples of mammals include humans, chimpanzees and other primates, pigs and horses, as well as birds such as ducks and chickens. Particularly preferred subjects for administration are humans.

The anti-nobecovirus agent according to the present embodiment may be administered to subjects for which Nobecovirus has been detected, not only during the period when symptoms are present, but also during the period when no clear symptoms are present, or during the incubation period after infection, for the purpose of preventing the onset of symptoms.

In another embodiment, the use of the above compounds for the production of the anti-nobecovirus agent is provided. In another embodiment, a method for treating, ameliorating, or preventing a disease caused by infection with Nobecovirus is provided. The method includes a step of administering the compounds to a subject.

In another embodiment, the above compound for use in treating a disease caused by infection with Nobecovirus is provided.

### Embodiment 4: Betacoronavirus proliferation inhibitor

The betacoronavirus proliferation inhibitor according to the present embodiment contains, as an active ingredient, a compound selected from the group consisting of ABT-737, A-1155463, gambogic acid, A-1331852, WEHI-539, and pharmacologically acceptable salts thereof. Below, differences between the betacoronavirus proliferation inhibitor and the anti-nobecovirus agent according to Embodiment 3 above are mainly described. For points not specifically mentioned, and regarding the betacoronavirus proliferation inhibitor, reference can be made to the description of the anti-nobecovirus agent according to Embodiment 3 above.

Examples of betacoronaviruses against which the betacoronavirus proliferation inhibitor is effective include Sarbecovirus, Nobecovirus, Merbecovirus and Embecovirus. Such Betacoronavirus for which the betacoronavirus proliferation inhibitor is effective may be one type or multiple types selected from the group consisting of Sarbecovirus, Nobecovirus, Merbecovirus and Embecovirus. For example, the Betacoronavirus may be one or more, two or more, three or more, or all four types selected from the group consisting of Sarbecovirus, Nobecovirus, Merbecovirus and Embecovirus. More specifically, the betacoronaviruses, for which the betacoronavirus proliferation inhibitor is effective, are SARS-CoV-1, SARS-CoV -2, Nobecovirus, MERS-CoV and Embecovirus. For example, the Betacoronavirus may be one or more, two or more, three or more, four or more, or all five types selected from the group consisting of SARS-CoV-1, SARS-CoV-2, Nobecovirus, MERS-CoV and Embecovirus.

The present inventors have found that navitoclax exhibits antiviral activity against multiple types of Betacoronavirus. As a result of further elucidation of the mechanism by which navitoclax produces a broad spectrum of anti-betacoronavirus activity, the present inventors have found that inhibition of Bcl-xL (b-cell lymphoma-extra large) is important. Based on these findings, the betacoronavirus proliferation inhibitor according to the present embodiment may contain a Bcl-xL expression inhibitor or a Bcl-xL inhibitor. In this case, the betacoronavirus proliferation inhibitor is particularly useful as a proliferation inhibitor for a betacoronavirus selected from the group consisting of Sarbecovirus, Merbecovirus, Nobecovirus and Embecovirus.

Examples of the Bcl-xL expression inhibitor include nucleic acids that inhibit the expression of Bcl-xL and vectors that express such nucleic acids. Bcl-xL is a mitochondrial transmembrane molecule. Bcl-xL is encoded by the BCL2L1 gene. Inhibiting the expression of Bcl-xL means to inhibit protein synthesis following the translation of mRNA, the transcript of the BCL2L1 gene. Examples of nucleic acids that inhibit the expression of Bcl-xL include siRNA (small interfering RNA), shRNA (small hairpin RNA), miRNA (micro RNA), and antisense nucleic acids that target a predetermined nucleotide sequence contained in the BCL2L1 gene as a target sequence. Here, the target sequence is a nucleotide sequence to which a nucleic acid molecule such as siRNA, miRNA, and antisense nucleic acid hybridizes.

The siRNA that inhibits the expression of the BCL2L1 gene is, for example, a double-stranded RNA comprising: the nucleotide sequence of mRNA encoding Bcl-xL or an RNA having a nucleotide sequence complementary to the target sequence; and its complementary strand.

The siRNA inhibits gene expression by RNA interference. In RNA interference, two RNA strands of siRNA hybridize to a target mRNA having a nucleotide sequence complementary to the nucleotide sequence, resulting in degradation of the target mRNA. Inhibition of gene expression by RNA interference has very high sequence specificity and can selectively inhibit the expression of target genes.

The target sequence and the nucleotide sequence complementary to the target sequence in siRNA are preferably completely complementary to each other. However, the nucleotide sequence in the siRNA may be the one that is not completely complementary to the target sequence and is allowed to undergo mutation, addition, and deletion of bases as long as at least 90% or more, preferably 95% or more sequence identity with the nucleotide sequence completely complementary to the target sequence. In particular, mutation, addition, or deletion of a base at an off-center position of the siRNA dose not completely eliminate the activity of degrading the target mRNA by RNA interference, and the degrading activity remains. On the other hand, in the case of mutations of bases located at or near the center of the siRNA, the effect is large and the activity of degrading the target mRNA by RNA interference is extremely reduced.

The length of the target sequence of siRNA is not particularly limited as long as it inhibits the expression of the BCL2L1 gene. The length of the target sequence is, for example, 18 or more, 19 or more, or 21 or more bases. When the length of siRNA is more than 23 to 26 or more bases, siRNA may be degraded by intracellular processing. Furthermore, considering the ease of siRNA synthesis, antigenicity issues, and transport to tissues *in vivo,* the length of the target sequence of siRNA is, for example, 50 or less bases, preferably 25 or less bases, preferably 19 or more bases and less than 25 bases, and more preferably 21 or more bases and less than 23 bases.

siRNA may have additional bases that do not form base pairs at the 5' and 3' ends. The length of the additional bases is not particularly limited as long as siRNA inhibits the expression of the BCL2L1 gene, but is 5 or less bases, for example, 2 or more bases and 4 or less bases. The additional bases can be either DNA or RNA, but using DNA can improve the stability of siRNA. Examples of such a sequence of additional bases include, but are not limited to, ug-3', uu-3', tg-3', tt-3', ggg-3', guuu- 3', gttt-3', ttttt-3', and uuuuu-3'.

The shRNA that inhibits the expression of the BCL2L1 gene is a single-stranded RNA wherein: the nucleotide sequence of mRNA encoding Bcl-xL or an RNA having a nucleotide sequence complementary to the target sequence contained in that nucleotide sequence; and its complementary strand are linked via a hairpin loop portion. shRNA forms a double-stranded structure by adopting the hairpin loop structure. The length of the hairpin loop portion is not particularly limited as long as shRNA inhibits the expression of the BCL2L1 gene, and is for example, 5 or more and 25 or less bases.

The miRNA that inhibits the expression of the BCL2L1 gene is, for example, a single-stranded RNA having the nucleotide sequence of mRNA encoding Bcl-xL or a nucleotide sequence complementary to the target sequence contained in that nucleotide sequence.

Like siRNA, miRNA inhibits the expression of the BCL2L1 gene. More specifically, miRNA forms a complex with multiple proteins and interacts with mRNA to which the BCL2L1 gene has been transcribed to inhibit gene expression.

The target sequence and a nucleotide sequence complementary to the target sequence in miRNA are preferably completely complementary to each other. However, the nucleotide sequence in miRNA does not have to be completely complementary to the target sequence, and may have at least 90% or more, preferably 95% or more sequence identity with the completely complementary nucleotide sequence.

The length of the target sequence of miRNA is not particularly limited as long as it inhibits the expression of the BCL2L1 gene. The length of the target sequence is preferably 18 or more bases and more preferably 20 or more bases. The length of the target sequence of miRNA is, for example, 50 or less bases, preferably 30 or less bases, and preferably 20 or more bases and less than 25 bases.

The antisense nucleic acid that inhibits the expression of the BCL2L1 gene is, for example, a nucleic acid having the nucleotide sequence of mRNA encoding Bcl-xL or a nucleotide sequence complementary to the target sequence of 20 or more consecutive bases contained in that nucleotide sequence. The antisense nucleic acid hybridizes to the target sequence of mRNA encoding Bcl-xL and thus to inhibit its expression.

The target sequence and the nucleotide sequence complementary to the target sequence in the antisense nucleic acid are preferably completely complementary to each other. However, the nucleotide sequence of the antisense nucleic acid does not have to be completely complementary to the target sequence, and may have at least 90% or more, preferably 95% or more sequence identity with the completely complementary nucleotide sequence.

The nucleic acid constituting the antisense nucleic acid may be DNA, RNA, or a hybrid of the two.

These siRNA, miRNA and antisense nucleic acid can be synthesized by known methods. The siRNA, miRNA and antisense nucleic acids are chemically synthesized by any nucleic acid synthesis method, such as a solid-phase phosphoramidite method. Further, siRNA, miRNA, and antisense nucleic acids can also be synthesized by a triester method. Various automated nucleic acid synthesizers are commercially available, and oligonucleotides can also be synthesized by such automated nucleic acid synthesizers. A multinucleotide synthesis method can also be used as appropriate.

The siRNA, miRNA, and antisense nucleic acids that inhibit the expression of the BCL2L1 gene can be prepared by determining the target sequence based on the nucleotide sequence of the BCL2L1 gene and synthesizing nucleic acids having a nucleotide sequence complementary thereto. For example, siRNA can be prepared by synthesizing RNA having a nucleotide sequence complementary to the target sequence and its complementary strand, respectively, in an automated nucleic acid synthesizer, maintaining them at about 90°C to 95°C for about 1 minute in an appropriate annealing buffer for denaturation, and then annealing at about 30°C to 70°C for about 1 to 8 hours.

Although siRNA, miRNA, and antisense nucleic acids can be synthesized from natural-type nucleic acids, phosphodiester bonds can be broken down in the case of natural-type nucleic acids by nucleolytic enzymes in cells. Accordingly, siRNA, miRNA, and antisense nucleic acids may be synthesized using modified nucleic acids such as thiophosphate and 2'-O-methyl types that are stable against degrading enzymes.

Vectors expressing nucleic acids that inhibit the expression of Bcl-xL express the above siRNA, shRNA, miRNA or antisense nucleic acids. With the use of the vector, the nucleotide sequence encoding the above siRNA, shRNA, miRNA or an antisense nucleic acid is ligated to a promoter that exhibits promoter activity in the cells of mammals or other organisms. The promoters used are not particularly limited as long as they can function in the cells.

In consideration of administration to mammals such as humans, viral vectors such as retrovirus, adenovirus, adeno-associated virus, Sendai virus, and herpes virus vectors are suitable. Adenoviruses have advantages such as extremely high gene transfer efficiency and the capability of being introduced into non-dividing cells. In adenoviruses, the expression of a transgene is transient and usually lasts only a few weeks, since incorporation of the transgene into the host chromosome is extremely rare. On the other hand, considering persistence, adeno-associated viruses, which have relatively high gene transfer efficiency, can be introduced into non-dividing cells, and are incorporated into chromosomes via an inverted terminal repeat sequence, are also preferred as vectors.

The above vectors preferably contain a transcription termination signal, specifically, a terminator region, downstream of the above siRNA, shRNA, miRNA or antisense-encoding nucleotide sequences. In addition, the above vector may further contain marker genes for selecting transformed cells (genes conferring resistance to drugs such as tetracycline, ampicillin, and kanamycin, genes complementing nutrient-requiring mutations, and the like.).

Examples of the Bcl-xL inhibitor include navitoclax, ABT-737, A-1155463, gambogic acid, A-1331852, WEHI-539 and pharmacologically acceptable salts thereof, aptamers designed against Bcl-xL, and anti-Bcl-xL antibodies.

The anti-Bcl-xL antibody can be prepared by immunizing animals with Bcl-xL or a Bcl-xL fragment as an antigen by known methods. Examples of target animals for immunization include mice, rats, rabbits, goats, and chickens. The anti-Bcl-xL antibody may be a polyclonal or monoclonal antibody. Polyclonal antibodies may be prepared by repeatedly injecting animals with antigens, and then purifying the blood collected after a predetermined period of time for the antibodies. Preferably, polyclonal antibodies are obtained by collecting blood from animals such as rabbits that have been sensitized with antigens and adjuvants, and then affinity purifying them with the antigens.

A monoclonal antibody is prepared by collecting B cells from the spleen or the lymph node of an animal immunized with an antigen, and then performing cell fusion to obtain a hybridoma. Then, hybridomas reacting with the antigen are selected and cloned. Thus, a monoclonal antibody can be produced by the hybridoma.

The anti-Bcl-xL antibody is not particularly limited, as long as it is an antibody that binds to Bcl-xL, and may be IgG, IgM, IgA, IgD or IgE, for example. Preferably, the anti-Bcl-xL antibody is IgG (IgG1, IgG2, IgG3 or IgG4). The anti-Bcl-xL antibody may be a full-length antibody or an antibody fragment (F(ab')2, Fab', Fab, Fv, single-stranded antibody, nanobody, scFv), as long as it can inhibit Bcl-xL functions.

The term "proliferation inhibition" of a virus as used herein means the inhibition of the replication of genomic RNA of the virus, preferably the inhibition of the replication of the genomic RNA within the host cell. Bcl-xL is known to inhibit apoptosis. For example, when a betacoronavirus proliferation inhibitor contains a Bcl-xL expression inhibitor or a Bcl-xL inhibitor, the genomic RNA replication is not inhibited as a result of apoptosis induced by the inhibition of Bcl-xL expression or the inhibition of Bcl-xL followed by phagocytosis of the host cells by immune cells, but is rather directly inhibited within the host cells.

As in Example 1 below, Bcl-xL is essential for the proliferation of Betacoronavirus, and a Bcl-xL inhibitor inhibits the proliferation of a wide range of betacoronaviruses. Therefore, in another embodiment, a pan-betacoronavirus inhibitor containing a Bcl-xL expression inhibitor or a Bcl-xL inhibitor is provided. The production method for the pan-betacoronavirus inhibitor, the ingredients other than the Bcl-xL expression inhibitor or the Bcl-xL inhibitor, the dosage, the administration route, and the like are the same as those for the anti-nobecovirus agent according to Embodiment 3 above.

In another embodiment, the use of the Bcl-xL expression inhibitor or the Bcl-xL inhibitor for the production of the pan-betacoronavirus inhibitor is provided. In another embodiment, a method for treating, ameliorating or preventing a disease caused by infection with Betacoronavirus is provided. The method includes a step of administering the Bcl-xL expression inhibitor or the Bcl-xL inhibitor to a subject. In another embodiment, the Bcl-xL expression inhibitor or the Bcl-xL inhibitor for use in treating a disease caused by infection with Betacoronavirus is provided.

Furthermore, as in Example 2 below, inhibition of Bcl-xL inhibits HCV replication. Thus, in another embodiment, an HCV proliferation inhibitor containing the Bcl-xL expression inhibitor or the Bcl-xL inhibitor is provided. In another embodiment, the use of the Bcl-xL expression inhibitor or the Bcl-xL inhibitor for the production of the HCV proliferation inhibitor is provided. In another embodiment, a method for treating, ameliorating, or preventing a disease caused by HCV proliferation is provided. The method includes administering the Bcl-xL expression inhibitor or the Bcl-xL inhibitor to a subject. In another embodiment, the Bcl-xL expression inhibitor or the Bcl-xL inhibitor for use in treating a disease caused by HCV proliferation is provided.

The Bcl-xL expression inhibitor or the Bcl-xL inhibitor inhibits the replication of a wide variety of viruses, since it inhibits HCV replication in addition to Betacoronavirus. For this reason, in another embodiment, a viral proliferation inhibitor containing the Bcl-xL expression inhibitor or the Bcl-xL inhibitor is provided. From the results of the cytotoxicity assessment in Example 1 below, the viral proliferation inhibitor inhibits viral replication at a concentration that is not cytotoxic. Therefore, the viral proliferation inhibitor can inhibit viral proliferation without relying on apoptosis of virus-infected cells. In another embodiment, the use of the Bcl-xL expression inhibitor or the Bcl-xL inhibitor for the production of a viral proliferation inhibitor is provided. In another embodiment, a method for treating, ameliorating, or preventing a disease caused by viral proliferation is provided. The method includes administering the Bcl-xL expression inhibitor or the Bcl-xL inhibitor to a subject. In another embodiment, the Bcl-xL expression inhibitor or the Bcl-xL inhibitor for use in treating a disease caused by viral proliferation is provided.

In another embodiment, a screening method for a therapeutic agent for a viral infectious disease is provided. The screening method for a therapeutic agent for a viral infectious disease includes a measurement step of measuring the Bcl-xL expression inhibitory activity or the Bcl-xL inhibitory activity of a test substance, and a selection step of selecting a test substance having Bcl-xL expression inhibitory activity or Bcl-xL inhibitory activity as a betacoronavirus proliferation inhibitor, a pan-betacoronavirus inhibitor, a viral proliferation inhibitor, or a hepatitis C virus proliferation inhibitor. The betacoronavirus proliferation inhibitor selected in the selection step is particularly useful as a proliferation inhibitor for two or more types of betacoronaviruses selected from the group consisting of Sarbecovirus, Merbecovirus, Nobecovirus, and Embecovirus.

Bcl-xL expression inhibitory activity in the measurement step may be determined by, for example, extracting, RNA in cells treated with a test substance, measuring the transcription amount of the BCL2L1 gene encoding Bcl-xL using a known technique such as quantitative PCR. Since Bcl-xL is an apoptosis inhibitor, for example, cells in which apoptosis has been induced may be treated with the test substance and the effect of the test substance on apoptosis may be evaluated in the measurement of Bcl-xL inhibitory activity. In this case, a test substance that enhances apoptosis compared to a control is a test substance having Bcl-xL inhibitory activity.

The above screening method for a therapeutic agent for a viral infectious disease can also be applied to screening for a pan-betacoronavirus inhibitor. Specifically, the screening method for a pan-betacoronavirus inhibitor includes a measurement step of measuring the Bcl-xL expression inhibitory activity or the Bcl-xL inhibitory activity of a test substance, and a selection step of selecting a test substance having Bcl-xL expression inhibitory activity or Bcl-xL inhibitory activity as a pan-betacoronavirus inhibitor.

Further, the above screening method for a therapeutic agent for a viral infectious disease can also be applied to screening for an HCV proliferation inhibitor. Specifically, the screening method for a HCV proliferation inhibitor includes a measurement step of measuring the Bcl-xL expression inhibitory activity or the Bcl-xL inhibitory activity of a test substance, and a selection step of selecting a test substance having Bcl-xL expression inhibitory activity or Bcl-xL inhibitory activity as an HCV proliferation inhibitor.

The present invention is described in more detail with reference to the following examples, but the present invention is not limited to these examples.

### Examples

### Example 1

### [Preparation of SARS-CoV-2 replicon]

### Preparation of BsaI(-) vector

Restriction enzyme *Bsa*I site (ggtctc) in an ampicillin resistance gene of pUC19 was subjected to gene transfer to be "gAtctc" using QuickChange mutagenesis (manufactured by Stratagene), thereby constructing a *Bsa*I-deficient plasmid pUC19b.

### Preparation of pSMART BACb

Restriction enzyme *Bsa*I site (gagacc) in the sopB gene of pSMART BAC was subjected to gene transfer to be "gagaTc" using QuickChange mutagenesis, thereby constructing a *Bsa*I-deficient plasmid, pSMART BACb.

### Preparation of cloning vector pSMART BACbc

pUC57 *Not*I-*Bsa*Ic*-Bsa*I-*Eco*RI-*Hind*III-*Bam*HI-*Xho*I-*Avr*II, into which an artificially synthesized multicloning site had been introduced, was cleaved with restriction enzymes *Not*I and *Avr*II to obtain an artificial gene of 955 bp represented by SEQ ID NO: 5. The artificial gene was ligated to the *Not*I*-Avr*II site of pSMART BACb to prepare pSMART BACbc.

### Preparation of cloning vector pHSG298c and pCC1-4kc

In order to introduce a *Bsa*I site into the pHSG298 vector, PCR fragments A and B containing *Bsa*I inside *Eco*RI and *Bam*HI were prepared. In preparation of PCR fragment A, PCR was performed using the HRP gene represented by SEQ ID NO: 6 as a template and a forward primer (SEQ ID NO: 7) and a reverse primer (SEQ ID NO: 8). The nucleotide sequence of PCR fragment A is represented by SEQ ID NO: 9. In preparation of PCR fragment B, PCR was performed using the HRP gene represented by SEQ ID NO: 6 as a template and a forward primer (SEQ ID NO: 10) and a reverse primer (SEQ ID NO: 11). The nucleotide sequence of PCR fragment B is represented by SEQ ID NO: 12.

Furthermore, in order to introduce a *Bsa*I site into the pCC1-4k vector, PCR fragment C containing *Bsa*I inside *Hind*III and *Bam*HI was prepared. In preparation of PCR fragment C, PCR was performed using the HRP gene represented by SEQ ID NO:6 as a template and a forward primer (SEQ ID NO:13) and a reverse primer (SEQ ID NO:14). The nucleotide sequence of PCR fragment C is represented by SEQ ID NO:15.

pHSG298 was cleaved with *EcoRI* and *BamHI,* and PCR fragment A was ligated to construct pHSG298c vector A. pHSG298 was also cleaved with *EcoRI* and *BamHI,* and PCR fragment B was ligated to construct pHSG298c vector B. pCCl-4k was cleaved with *Hind*III and *Bam*HI*,* and PCR fragment C was ligated to construct a pCC1-4kc vector.

### Design of artificial gene

Without using a natural virus as a template, a SARS-CoV-2 replicon (hereinafter referred to as "SC2R") having the nucleotide sequence represented by SEQ ID NO: 16 was artificially synthesized based on the genetic information of SARS-CoV-2 (NCBI Reference Sequence: NC_045512) on the database of the National Center for Biotechnology Information (NCBI) as follows. SC2R contains a CMV promoter sequence, SARS-CoV-2 non-structural regions (ORF1a and ORF1b), sNLuc, Neo, N gene, pA, Rz, and BGH from the 5' end, similar to the Nobecovirus replicon DNA illustrated in FIG. 1B. Note that the nucleotide sequence corresponding to the *Bsa*I site (gagacc) in the non-structural region of SC2R was designated as "gGgacc" to perform synonymous substitution.

SC2R (25,562 bp) in which a *Bsa*I site had been substituted was divided into 10 fragments F1 to F10 of 2 kb to 3 kb to synthesize artificial genes. In detail, the 2,735 bp ranging from 1 to 2735th bases from the 5' end of the nucleotide sequence of SC2R represented by SEQ ID NO: 16 was designated as F1, the 2,829 bp ranging from 2,732 to 5,360th bases from the same was designated as F2, the 2,795 bp ranging from 5,357 to 8,151th bases from the same was designated as F3, the 2,446 bp ranging from 8,148 to 10,593th bases from the same was designated as F4, the 2,790 bp ranging from 10,590 to 13,379th bases from the same was designated as F5, the 2,502 bp ranging from 13,376 to 15,877th bases from the same was designated as F6, the 2,835 bp ranging from 15,874 to 18,708th bases from the same was designated as F7, the 2,410 bp ranging from 18,705 to 21,114th bases from the same was designated as F8, the 2,178 bp ranging from 21,111 to 23,288th bases from the same was designated as F9, and the 2,278 bp ranging from 23,285 to 25,562nd bases from the same was designated as F10. The nucleotide sequence of the *Bsa*I site was added at both ends of each fragment, allowing SC2R to be seamlessly integrated into pSMART BACbc.

### Synthesis of artificial genes

A *Bsa*I site was introduced at the 5' and 3' ends of each of the 10 fragments. F1 to F4 and F6 to F10 were introduced into pUC19b (pUC19b/SC2R-F1, F2, F3, F4, F6, F7, F8, F9, F10). F5 was introduced into the pCC1-4k vector (pCCl-4k/SC2R-F5).

### Preparation of intermediate fragments pHSG298c/F123, pCCl-4kc/F456 and pHSG298c/F7-10

pUC19b/F1, F2, F3, F4, F6, F7, F8, F9, F10 and pCC1-4k/F5 were each cleaved with *Bsa*I. F1, F2 and F3 were introduced into pHSG298c vector A to give pHSG298c/F123. F4, F5 and F6 were introduced into the pCCl-4kc vector to give pCC1-4kc/F456. F7, F8, F9 and F10 were introduced into pHSG298c vector B to give pHSG298c/F7-10.

### Preparation of SC2R vector

pHSG298c/F123, pCC1-4kc/F456 and pHSG298c/F7-10 were cleaved with *Bsa*I, and each fragment was ligated to the *Bsa*I-*Bsa*I site of pSMART BACbc. After ligation, the resultants were introduced into BAC-Optimized Replicator v2.0 Electrocompetent Cells (manufactured by Lucigen) by electroporation for cloning. As a result, pSMART BACbc/SC2R was obtained.

### [Preparation of SARS-CoV-1 replicon, MERS-CoV replicon and Nobecovirus replicon]

### Design of artificial gene

The SARS-CoV-1 replicon (hereinafter referred to as "SCVR"), MERS-CoV replicon (hereinafter referred to as "MERVR") and Nobecovirus replicon (hereinafter referred to as "NOBEVR"), whose nucleotide sequences are represented by SEQ ID NOS: 17, 18 and 1, respectively, were prepared by artificial synthesis based on the genetic information of each virus on the NCBI database (SARS-CoV-1: NC_004718, MERS-CoV: NC_019843, Nobecovirus: NC_009021) as follows. SCVR, MERVR and NOBEVR also contain, from the 5' end, the CMV promoter sequence, the non-structural regions (ORF1a and ORF1b) of each virus, sNLuc, Neo, N gene, pA, Rz and BGH. SCVR, MERVR and NOBEVR were artificially synthesized in their entirety (one fragment).

### Preparation of SCVR, MERVR and NOBEVR vectors

For each of SCVR, MERVR and NOBEVR, a full-length fragment in which a*Bsa*I site had been introduced at each of the 5'-end and 3'-end was ligated to the *Bsa*I-*Bsa*I site of pSMART BACbc. After ligation, cloning was performed in the same manner as for SC2R to obtain pSMART BACbc/SCVR, pSMART BACbc/MERVR and pSMART BACbc/NOBEVR.

### [Preparation of hibecovirus replicon and embecovirus replicon]

### Artificial gene design

The Hibecovirus replicon (hereinafter referred to as "HibeCoVR") and the Embecovirus replicon (hereinafter referred to as "HKU1"), whose nucleotide sequences are represented by SEQ ID NOS: 19 and 20, respectively, were prepared by artificial synthesis based on the genetic information of each virus on the NCBI database (HibeCoVR: NC_0252 12, HKU1: NC_006577) as follows. Similar to SC2R, HibeCoVR and HKU1 also contain, from the 5' end, the CMV promoter sequence, the non-structural regions (ORF1a and ORF1b) of each virus, sNLuc, Neo, the N gene, pA, Rz, and BGH.

### Synthesis of artificial genes

HibeCoVR was artificially synthesized by dividing it into 10 fragments (F1 to F10). *Bsa*I sites were introduced at both the 5' and 3' ends of each of the divided fragments. Each fragment was introduced into pUC19b, and cloned using *E. coli* DH5 strain (manufactured by TOYOBO CO., LTD.) (pUC19b/hib-F1, F2, F3, F4, F6, F7, F8, F9, F10). The 2,601 bp ranging from 1 to 2,601th bases from the 5' end of the nucleotide sequence of HibeCoVR represented by SEQ ID NO: 19 was designated as F1, the 2,833 bp ranging from 2,598 to 5,430th bases from the same was designated as F2, the 2,940 bp ranging from 5,427 to 8,366th bases from the same was designated as F3, the 2,813 bp ranging from 8,363 to 11,175th bases from the same was designated as F4, the 2,651 bp ranging from 11,172 to 13,822nd bases from the same was designated as F5, the 2,730 bp ranging from 13,819 to 16,548th bases from the same was designated as F6, the 2,144 bp ranging from 16,545 to 18,688th bases from the same was designated as F7, the 2,856 bp ranging from 18,685 to 21,540th bases from the same was designated as F8, the 2,532 bp ranging from 21,537 to 24,068th bases from the same was designated as F9, and the 2,152 bp ranging from 24,065 to 26,212th bases from the same was designated as F10.

HKU1 was artificially synthesized by dividing it into nine fragments (F1 to F9). *Bsa*I sites were introduced at both the 5' and 3' ends of each of the divided fragments. Each fragment was introduced into pUC19b and cloned using *E. coli* DH5 strain (manufactured by TOYOBO CO., LTD.) (pUC19b/hku-F1, F2, F3, F4, F6, F7, F8, F9). The 2,901 bp ranging from 1 to 2901th bases from the 5' end of the nucleotide sequence of HKU1 represented by SEQ ID NO:20 was designated as F1, the 2,604 bp ranging from 2,898 to 5,501st bases from the same was designated as F2, the 2,804 bp ranging from 5,498 to 8,301st bases from the same was designated as F3, the 2,704 bp ranging from 8,298 to 11,001st bases from the same was designated as F4, the 2,954 bp ranging from 10,998 to 13,951st bases from the same was designated as F5, the 2,644 bp ranging from 13,948 to 16,591st bases from the same was designated as F6, the 2,864 bp ranging from 16,588 to 19,451st bases from the same was designated as F7, the 2,918 bp ranging from 19,448 to 22,365th bases from the same was designated as F8, and the 3,385 bp ranging from 22,362 to 25,746th bases from the same was designated as F9.

### Preparation of cloning vector pHSG298c and intermediate fragments

PCR fragments D, E, F and G containing *Bsa*I inside *EcoRI* and *BamHI* were prepared. In preparation of PCR fragment D, PCR was performed using the HRP gene represented by SEQ ID NO: 6 as a template and a forward primer (SEQ ID NO: 21) and a reverse primer (SEQ ID NO: 14). The nucleotide sequence of PCR fragment D is represented by SEQ ID NO: 22. In preparation of PCR fragment E, PCR was performed using the HRP gene represented by SEQ ID NO: 6 as a template and a forward primer (SEQ ID NO: 23) and a reverse primer (SEQ ID NO: 24). The nucleotide sequence of PCR fragment E is represented by SEQ ID NO: 25. In preparation of PCR fragment F, PCR was performed using the HRP gene represented by SEQ ID NO: 6 as a template and a forward primer (SEQ ID NO: 26) and a reverse primer (SEQ ID NO: 27). The nucleotide sequence of PCR fragment F is represented by SEQ ID NO: 28. In preparation of PCR fragment G, PCR was performed using the HRP gene represented by SEQ ID NO: 6 as a template and a forward primer (SEQ ID NO: 29) and a reverse primer (SEQ ID NO: 14). The nucleotide sequence of PCR fragment G is represented by SEQ ID NO: 30.

pHSG298 was cleaved with *EcoRI* and *BamHI,* and PCR fragments D, E, F, and G were ligated to construct pHSG298c vectors D, E, F, and G, respectively.

pUC19b/hib-F1, F2, and F3 cleaved with *Bsa*I were introduced into the above pHSG298c vector A to obtain pHSG298c/hib-F123. pUC19b/hib-F4, F5, and F6 cleaved with *Bsa*I were introduced into pHSG298c vector D to obtain pHSG298c/hib-F456. pUC19b/hib-F7 and F8 cleaved with *Bsa*I were introduced into the above pHSG298c vector B to obtain pHSG298c/hib-F78. pUC19b/hib-F9 and F10 cleaved with *Bsa*I were introduced into pHSG298c vector E to obtain pHSG298c/hib-F910.

pUC19b/hku-F1, F2, and F3 cleaved with *Bsa*I were introduced into pHSG298c vector F to obtain pHSG298c/hku-F123. pUC19b/hku-F4, F5, and F6 cleaved with *Bsa*I were introduced into pHSG298c vector G to obtain pHSG298c/hku-F456. pUC19b/hku-F7, F8, and F9 cleaved with *Bsa*I were introduced into the above-mentioned pHSG298c vector B to obtain pHSG298c/hku-F789.

### Preparation of HibeCoVR vector

Each of pHSG298c/hib-123, pHSG298c/hib-F456, pHSG298c/hib-F78 and pHSG298c/hib-F910 was cleaved with *Bsa*I, and each fragment was ligated to the *Bsa*I-*Bsa*I site of pSMART BACbc. After ligation, cloning was performed in the same manner as for SC2R to obtain pSMART BACbc/HibeCoVR.

### Preparation of HKU1 vector

Each of pHSG298c/hku-123, pHSG298c/hku-F456 and pHSG298c/hku-F789 was cleaved with *Bsa*I, and each fragment was ligated to the *Bsa*I-*Bsa*I site of pSMART BACbc. After ligation, cloning was performed in the same manner as for SC2R to obtain pSMART BACbc/HKU1.

### Establishment of HuH-7.6c cells

When G418 was added after introducing reporter full-length HCV RNA into HuH-7 cells by electroporation, cells with a high HCV RNA replication level were selected to form colonies. An OR6 cell line was established as a cell line from the cloned cells with the highest HCV RNA replication level among the colonies. Next, IFN was added to the OR6 cells to eliminate HCV RNA, and thus a cured cell line (HuH-7.6c cell line) was established. It is known that the cured cell line has a higher replication level of HCV RNA than the parental HuH-7 cell line (Masanori Ikeda and 5 others, "Efficient replication of a full-length hepatitis C virus genome, strain O, in cell culture, and development of a luciferase reporter system", Biochem Biophys Res Commun., 2005, 329(4):1350-9).

### Evaluation of anti-betacoronavirus activity

HuH-7.6c cells were seeded in a 6-well plate at 1 x 10⁵ cells/well, and on the next day, 1 µg of pSMART BACbc/SC2R, pSMART BACbc/SCVR, pSMART BACbc/MERVR, pSMART BACbc/NOBEVR, pSMART BACbc/HibeCoVR or pSMART BACbc/HKU1 was transfected into the cells using Fugene HD reagent. After 24 hours, the compound was added at a predetermined concentration. After 48 hours of culture, the culture supernatant was collected and luciferase activity was measured using the Nano-Glo Luciferase Assay System.

Two (2) µg of pSMART BACbc/SC2R was introduced into OR6c cells derived from HuH-7 cells by lipofection. After 24 hours, the cells were subcultured onto a 24-well plate and cultured for 24 hours, followed by addition of A-1331852 at a predetermined concentration. Forty eight (48) hours after the addition of A-1331852, the cells were collected and the intracellular luciferase activity was measured. The 50% inhibitory concentration (EC₅₀) was calculated assuming that the luciferase activity of cells to which no A-1331852 had been added was 100%.

### Evaluation of cytotoxicity

HuH-7.6c cells were seeded in a 96-well plate at 5 x 10³ cells/well. 24 hours after the start of culture, the compound was added to the medium at a predetermined concentration. After 48 hours of culture, 10 µl of Premix WST-1 Cell Proliferation Assay System (Takara Bio) was added to the medium, and then cells were cultured at 37°C for 2 hours, after which the absorbance at 450 nm was measured using a microplate reader.

### Knockdown of Bcl-2 family by siRNA

HuH-7.6c cells were seeded in a 6-well plate at 1 x 10⁵ cells/well. After 24 hours of culture, the concentration of each siRNA (siBCL2) against the BCL2 gene encoding Bcl-2, siRNA (siBCL2L1) against the BCL2L1 gene encoding Bcl-xL, and siRNA (siBCL2L2) against the BCL2L2 gene encoding Bcl-w (all manufactured by Dharmacon Inc.) was adjusted at a final concentration of 40 nM and introduced into the cells using Lipofectamine (trademark) RNAiMAX reagent (manufactured by Thermo Fisher Scientific Inc.). After 24 hours, 2 µg of pSMART BACb/SC2R was transfected into the cells using Fugene HD reagent (manufactured by Promega corporation). After 24 hours, the cells were detached using trypsin and seeded in a 24-well plate at 3 x 10⁴ cells/well. After 48 hours of culture, the culture supernatant was collected and luciferase activity was measured using the Nano-Glo Luciferase Assay System (Promega Corporation).

### [Result]

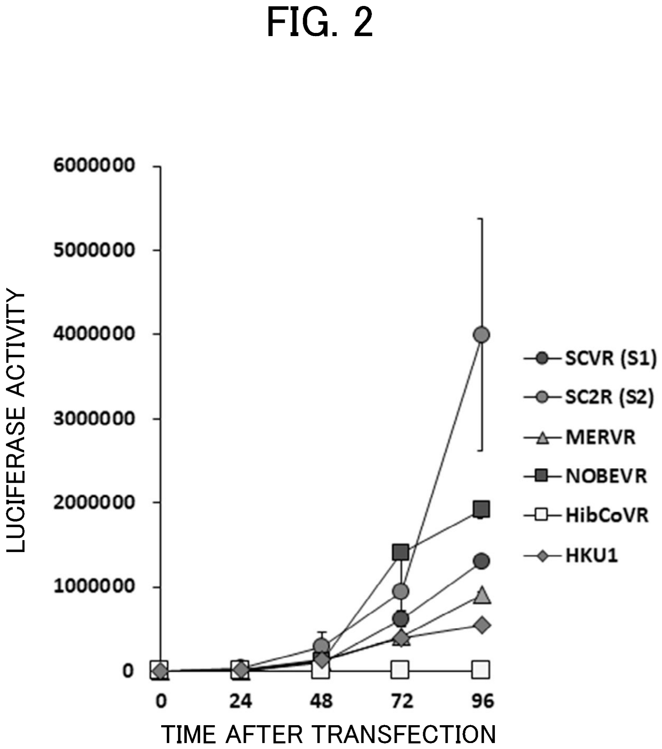

As illustrated in FIG. 2, replication of replicon RNAs transcribed from SC2R, SCVR, MERVR, NOBEVR, and HKU1, except for HibeCoVR, was confirmed in HuH-7.6c cells.

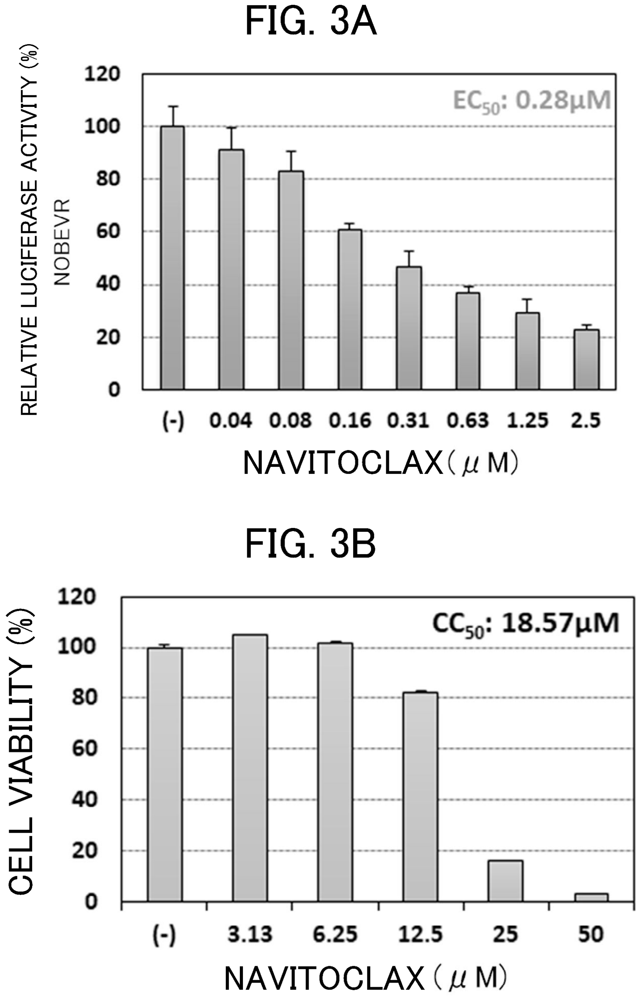

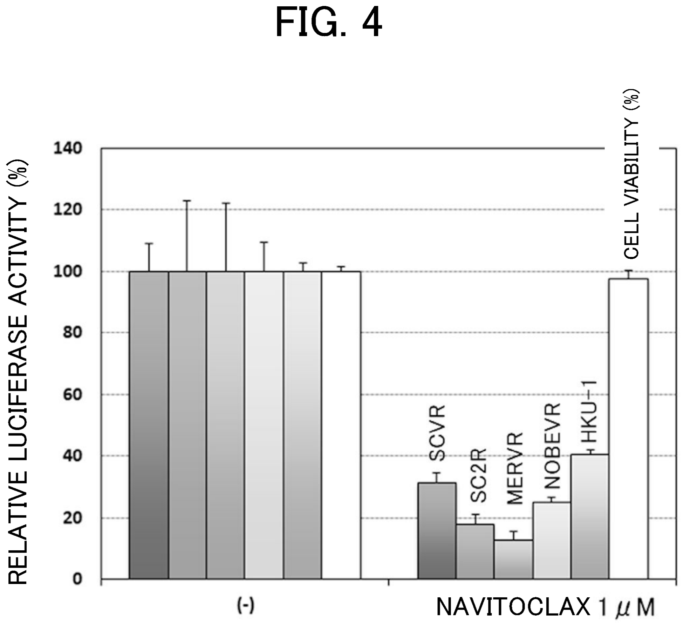

As illustrated in FIG. 3A, navitoclax inhibited the replication of replicon RNA transcribed from NOBEVR in a concentration-dependent manner (EC₅₀ = 0.28 µM). FIG. 3B illustrates the viability of cells exposed to navitoclax. The cytotoxicity (CC₅₀) of navitoclax was 18.57 µM. As illustrated in FIG. 4, navitoclax (1 µM) inhibited the replication of replicon RNA transcribed from SC2R, SCVR, MERVR, NOBEVR and HKU1.

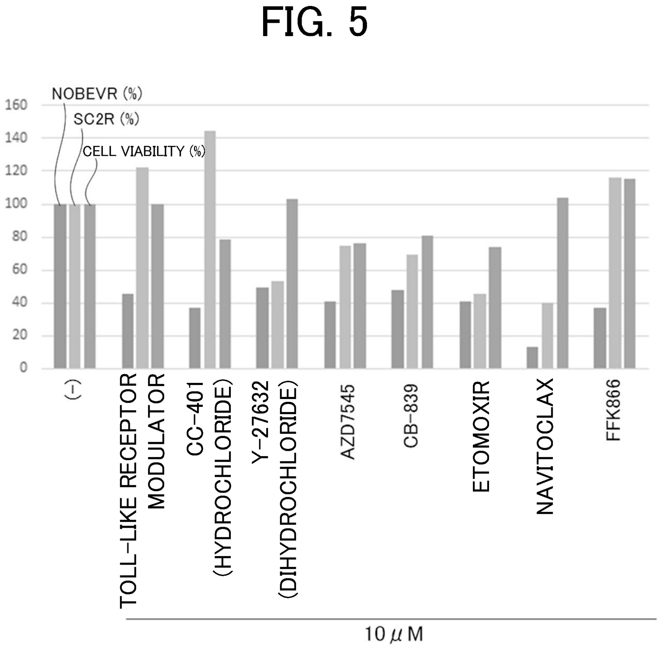

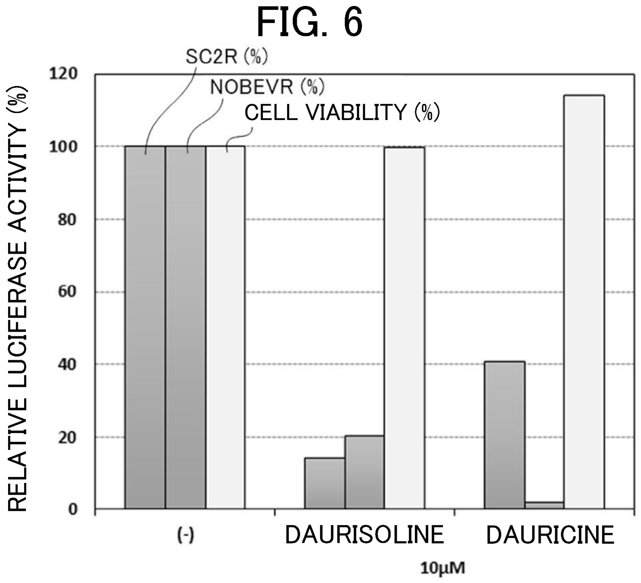

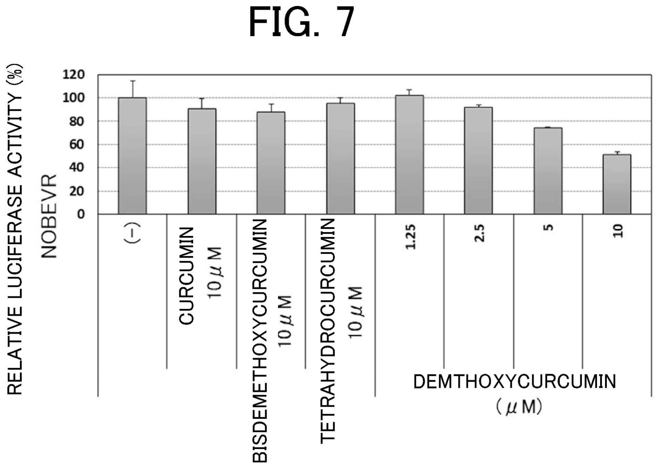

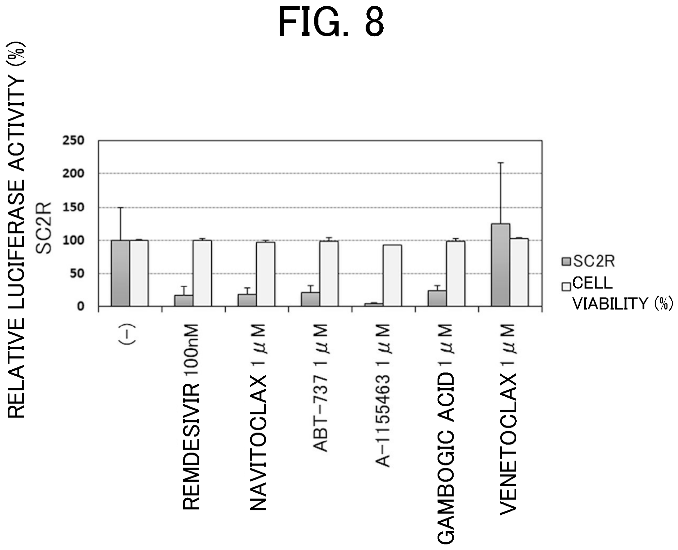

350 types of compounds from the Phase II drop library of MedChemexpress and natural product library were screened for compounds that inhibit the replication of replicon RNA transcribed from NOBEVR. Regarding the Phase II drop library, as illustrated in FIG. 5, 8 types of compounds (10 µM) including navitoclax were found to have inhibitory activity against the replication of replicon RNA transcribed from NOBEVR. As illustrated in FIGS. 6 and 7, daurisoline, dauricine, and demethoxycurcumin from the natural product library were confirmed to have strong inhibitory activity against the replication of replicon RNA transcribed from NOBEVR. Daurisoline and dauricine also had replicon RNA replication inhibitory activity against SC2R.

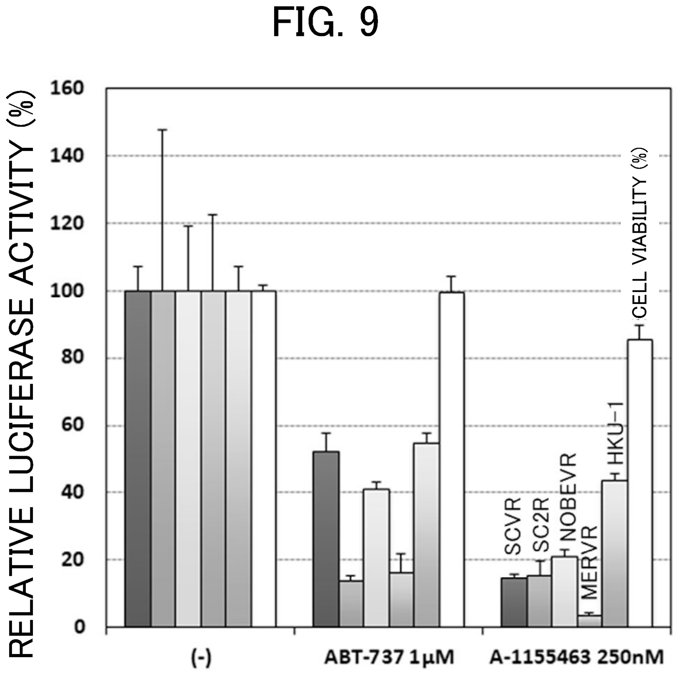

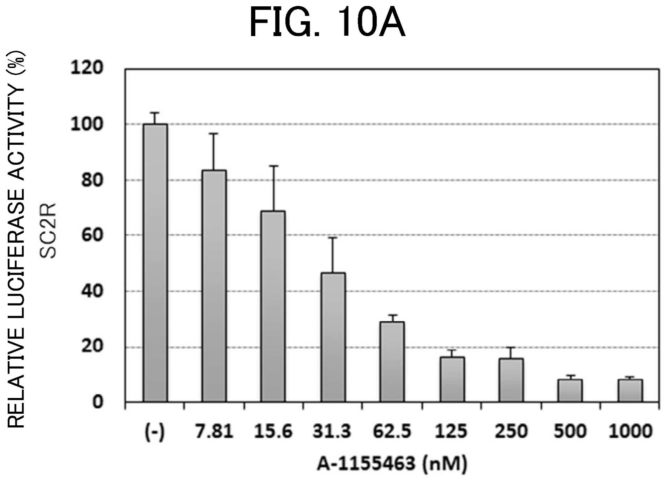

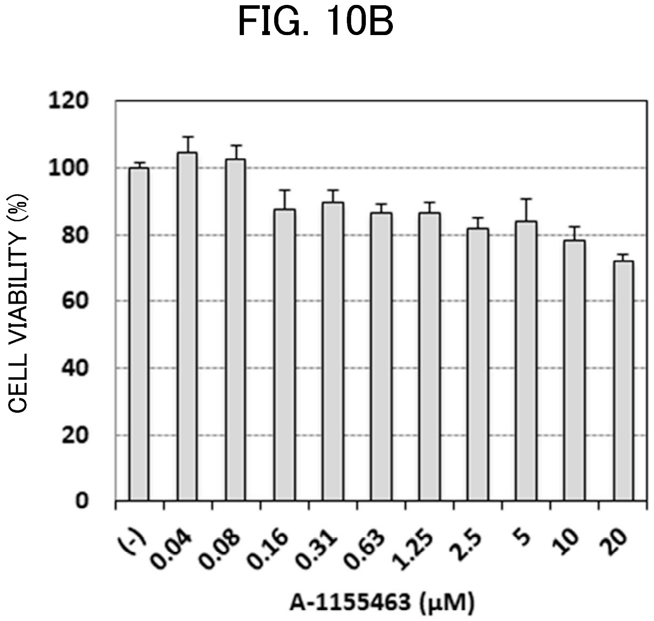

Navitoclax has inhibitory activity against Bcl-2, Bcl-w and Bcl-xL. Therefore, the anti-betacoronavirus activity of Bcl-2, Bcl-w and Bcl-xL inhibitors was examined. As illustrated in FIG. 8, venetoclax, a Bcl-2 selective inhibitor, did not inhibit the replication of replicon RNA transcribed from SC2R. On the other hand, ABT-737, A-1155463 and gambogic acid including navitoclax, which have inhibitory activity against Bcl-xL, inhibited the replication of replicon RNA transcribed from SC2R. As illustrated in FIG. 9, ABT-737 and A-1155463 also inhibited the replication of replicon RNA transcribed from SCVR, MERVR, NOBEVR and HKU1. As illustrated in FIG. 10A, A-1155463 inhibited the replication of replicon RNA transcribed from SC2R in a concentration-dependent manner. FIG. 10B illustrates the viability of cells exposed to A-1155463.

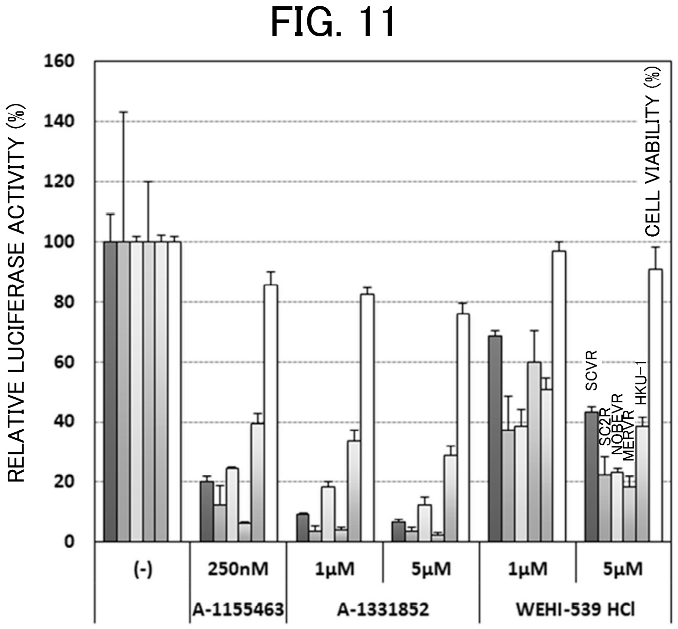

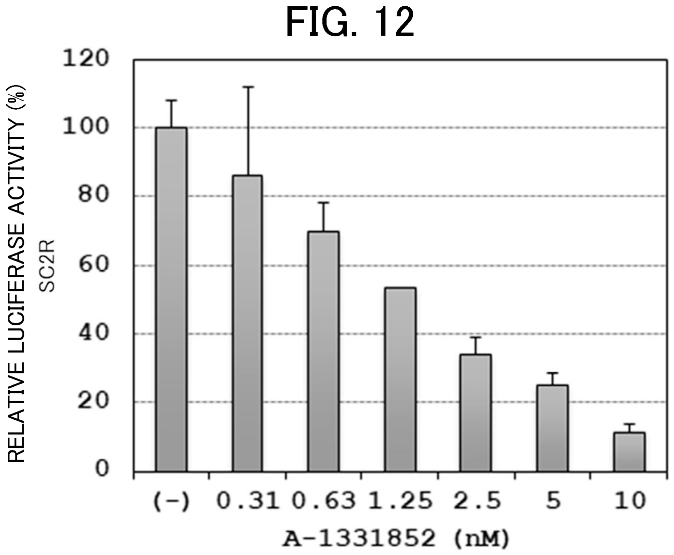

As illustrated in FIG. 11, Bcl-xL selective inhibitors, A-1155463, A-1331852 and WEHI-539 HCl inhibited the replication of replicon RNA transcribed from SC2R, SCVR, MERVR, NOBEVR and HKU1. In the evaluation using OR6c cells, A-1331852 inhibited the replication of replicon RNA transcribed from SC2R in a concentration-dependent manner (EC₅₀ = 1.46 nM) as illustrated in FIG. 12.

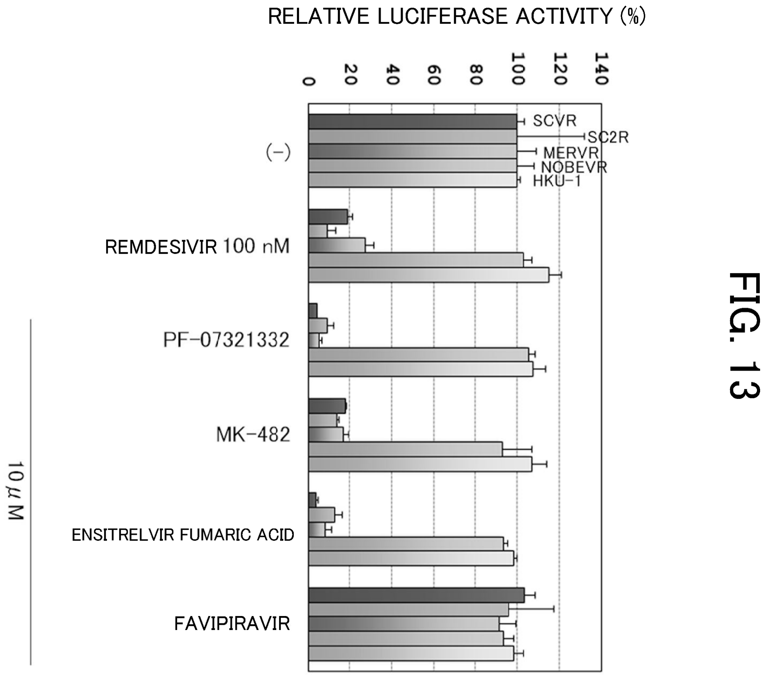

The results of evaluating the effects of existing drugs against COVID-19, remdesivir (100 nM), PF-07321332 (10 µM), MK-482 (10 µM), ensitrevir fumarate (10 µM) and favipiravir (10 µM) on the replication of replicon RNA transcribed from SC2R, SCVR, MERVR, NOBEVR and HKU1 are illustrated in FIG. 13. Existing drugs did not inhibit the replication of replicon RNA in NOBEVR and HKU1. It was demonstrated that existing therapeutic agents for COVID-19 are ineffective in the case of an outbreak of a nobecovirus that has not been reported to infect humans.

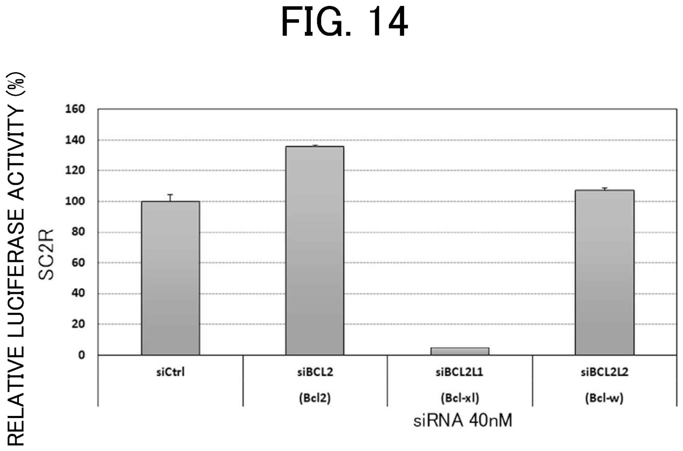

As illustrated in FIG. 14, knocking down the BCL2L1 gene with siRNA inhibited the replication of replicon RNA transcribed from SC2R.

### Example 2

### [Preparation of HCV replicons]

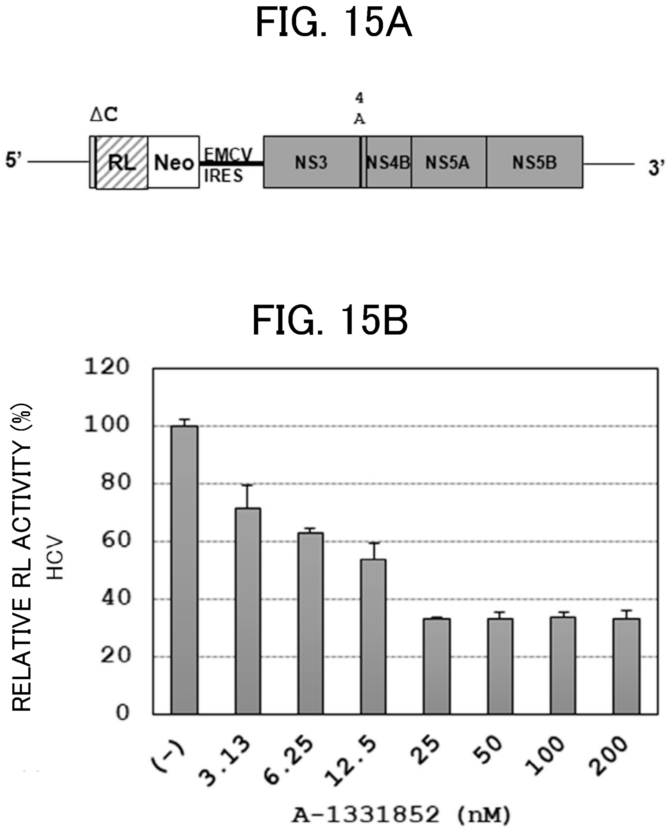

The antiviral activity of A-1331852 against HCV was evaluated. For the evaluation system, s1B-4R cells (Antiviral Research, 82, 2009, 42-50) in which the HCV subgenomic replicon replicates were used. s1B-4R cells are of a cell line in which the subgenomic replicon of genotype 1b HCV (1B-4 strain) stably replicates in HuH-7 cells. As illustrated in FIG. 15A, the HCV subgenomic replicon has Renilla luciferase (RL) and neomycin resistance gene (Neo) on the 5' side. The non-structural region (NS3-NS4A-NS4B-NS5A-NS5B) required for HCV replication is translated by the IRES of encephalomyocarditis virus (EMCV).

To construct the HCV replicon, pRN/3-5B/KE plasmid (Biochem. Biophys. Res. Commun., 329, 2005, 1350-1359) was used as a cassette vector. The non-structural region was substituted with reverse transcription PCR products of serum containing the 1B-4 strain with *Spe*I of NS3 and BsiWI of NS5B. The PCR products were further amplified with primers NS3 *Spe*I (SEQ ID NO: 31) containing an *Spe*I site and NS5B *BsiWI* (SEQ ID NO: 32) containing a *BsiWI* site. The amplified product was cleaved with *Spe*I and *BsiWI,* and the resulting fragment was inserted into a pRN/3-5B/KE plasmid that had been previously cleaved with *Spe*I and *BsiW*I.

Plasmid DNA was linearized with *Xba*I and used for RNA synthesis with T7 MEGAscript (Ambion). 10 µg of the synthesized HCV replicon RNA was introduced into OR6c cells derived from HuH-7 cells by electroporation and selected for 3 weeks in the presence of G418 (0.3 mg/ml).

A-1331852 at a predetermined concentration was added to cells transfected with the HCV replicon, and the cells were collected 72 hours later and RL activity was measured.

### [Result]

As illustrated in FIG. 15B, A-1331852 inhibited the replication of HCV replicon RNA in a concentration-dependent manner (EC₅₀ = 14.8 nM). Since the Bcl-xL inhibitor exhibited antiviral activity against HCV in addition to betacoronaviruses, it is considered to be effective against a wide range of viruses.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

The present application is based on Japanese Patent Application No. 2023-064237, filed on April 11, 2023. The entire description, claims, and drawings of Japanese Patent Application No. 2023-064237 are incorporated herein by reference.

### Industrial Applicability

The present disclosure is suitable for screening for an anti-nobecovirus agent, a betacoronavirus proliferation inhibitor, a pan-betacoronavirus inhibitor, a viral proliferation inhibitor, a hepatitis C virus proliferation inhibitor, and an anti-nobecovirus agent, and screening for a therapeutic agent for a viral infectious disease.

## Claims

1. A replicon DNA, comprising:
a promoter sequence that functions in a vertebrate cell;
a non-structural region comprising nobecovirus non-structural protein genes 1a and 1b located on the 3' side of the promoter sequence; and
a nobecovirus N gene located on the 3' side of the non-structural region.

2. The replicon DNA according to claim 1, further comprising a reporter gene.

3. A cloning vector, comprising the replicon DNA according to claim 1 or 2.

4. A screening kit, comprising:
the cloning vector according to claim 3; and
a hepatitis C virus-cured cell.

5. A screening method, comprising:
exposing a hepatitis C virus-cured cell transfected with the cloning vector according to claim 3 to a test substance;
quantifying a replicon RNA replicated from a replicon RNA transcribed from the replicon DNA in the hepatitis C virus-cured cell; and
selecting a test substance that inhibits the replication of the replicon RNA more than a control.

6. An anti-nobecovirus agent, comprising a compound selected from the group consisting of navitoclax, ABT-737, A-1 155463, gambogic acid, A-1331852, WEHI-539, toll-like receptor modulator, CC-401, Y-27632, AZD7545, CB-839, etomoxir, FK-866, daurisoline, daurisine, demethoxycurcumin and a pharmacologically acceptable salt thereof.

7. The anti-nobecovirus agent according to claim 6, which is a nobecovirus proliferation inhibitor.

8. A betacoronavirus proliferation inhibitor, comprising a Bcl-xL expression inhibitor or a Bcl-xL inhibitor.

9. A betacoronavirus proliferation inhibitor, comprising a compound selected from the group consisting of ABT-737, A-1155463, gambogic acid, A-1331852, WEHI-539 and a pharmacologically acceptable salt thereof.

10. The betacoronavirus proliferation inhibitor according to claim 8 or 9, wherein the betacoronavirus is one or more types of betacoronaviruses selected from the group consisting of Sarbecovirus, Merbecovirus, Nobecovirus, and Embecovirus.

11. A pan-betacoronavirus inhibitor, comprising a Bcl-xL expression inhibitor or a Bcl-xL inhibitor.

12. A viral proliferation inhibitor, comprising a Bcl-xL expression inhibitor or a Bcl-xL inhibitor.

13. A hepatitis C virus proliferation inhibitor, comprising a Bcl-xL expression inhibitor or a Bcl-xL inhibitor.

14. A screening method for a therapeutic agent for a viral infectious disease, comprising:
measuring the Bcl-xL expression inhibitory activity or the Bcl-xL inhibitory activity of a test substance; and
selecting the test substance having the Bcl-xL expression inhibitory activity or the Bcl-xL inhibitory activity as a proliferation inhibitor for two or more types of betacoronaviruses selected from the group consisting of Sarbecovirus, Merbecovirus, Nobecovirus, and Embecovirus, a pan-betacoronavirus inhibitor, a viral proliferation inhibitor, or a hepatitis C virus proliferation inhibitor.
